# EUROPEAN PATENT APPLICATION

(11) **EP 2 006 011 A1**
(43) Date of publication of application: **24.12.2008**
(21) Application number: 07110840.1
(22) Date of filing: 22.06.2007
(51) Int. Cl.: B01D 53/04

(54) **Process for reducing carbon monoxide in olefin-containing hydrocarbon feedstocks**

(71) Applicant: TOTAL PETROCHEMICALS RESEARCH FELUY, 7181 Seneffe (Feluy) (BE)
(72) Inventor: Gauthier, Bill, B-1560, Hoeilaart (BE); Hortmann, Kai, B-1700, Dilbeek (BE); Vandewiele, David, B-7110, Strépy-Bracquegnies (BE)
(74) Representative: Leyder, Francis

(57) **Abstract**

A process for removing carbon monoxide from an olefin-containing hydrocarbon feedstock comprising the steps of: a) passing the said hydrocarbon feedstock over a material comprising nickel deposited on a support material wherein said nickel is present as both nickel oxide and metallic nickel; and b) recovering a hydrocarbon stream having a substantially reduced carbon monoxide content.

## Description

### FIELD OF THE INVENTION

The present invention relates to a process for the reduction of carbon monoxide in olefin-containing hydrocarbons. More particularly, the present invention relates to a process for the reduction of carbon monoxide in propylene feedstocks.

### BACKGROUND OF THE INVENTION

As is well known, olefins, in particular ethylene and propylene, are used to produce numerous types of intermediate and end products, which are predominantly polymeric materials. Commercial production of olefins is almost entirely carried out by thermal cracking of hydrocarbon feedstocks containing ethane, propane, liquid naphtha or mixtures thereof. Such feedstocks may also have significant amounts of impurities, such as acetylenic compounds, carbonylsulfide, arsine, carbon dioxide, and carbon monoxide.

Acetylenic impurities, such as methylacetylene in the case of propylene feedstocks, are most commonly removed by selective hydrogenation in the presence of a hydrogenation catalyst and hydrogen. However, not only is the reaction highly exothermic, but also the rate of conversion of olefins to paraffins is 100 times faster than that of acetylenes to olefins, for example, methylacetylene to propylene. However, since hydrogenation catalysts generally have higher adsorbtivity of carbon monoxide than of vinylic compounds, thermal runaway of the reaction and undesirable olefin hydrogenation can be avoided by having relatively high concentrations of carbon monoxide in the original feedstock. Hence olefin feedstocks obtained from thermal cracking and acetylenic hydrogenation may have large amounts of carbon monoxide. Even after cryogenic distillation, these impurities may still be present, ranging from 0.01 to 100 parts per million (ppm) by weight.

Other typical processes to obtain olefins, such as fluid catalytic cracking and MTO ("methanol to olefins") conversion, may also give rise to feedstocks having high and fluctuating amounts of carbon monoxide.

These same olefins are subsequently catalytically converted to a multitude of polymeric products on a large scale. Various types of catalysts can be used for the polymerisation process. Due to improved product qualities, metallocene catalysts are becoming increasingly prevalent in industry. Unfortunately, these new generation catalysts are, as well as being much more expensive, also very sensitive. Their activities are severely limited by impurities present in the hydrocarbon or hydrogen feed. It is well known that carbon monoxide is an extremely strong poison for metallocene catalysts. If the concentration of carbon monoxide is more than 5-20 parts per billion (ppb) by weight, the activity of the metallocene catalyst will be adversely affected.

Other processes besides polymerisations also require purified hydrocarbons. For example, US 2005/0137443 describes the need of carbon monoxide removal from olefin-containing hydrocarbon feed, because catalysts used for paraffin isomerisation are also highly carbon monoxide sensitive. US 2005/0247196 describes the need of carbon monoxide elimination from hydrocarbon gas to make low-level calibration standards.

Several methods are known in the art, which attempt to separate carbon monoxide from gaseous mixtures. These include, for instance, distillation, liquid adsorption and membrane separation. For a man skilled in the art, the obvious solution to remove carbon monoxide from olefin-containing hydrocarbon feedstocks is by distillation, as carbon monoxide's boiling point of -192°C is far below that of any olefin and therefore easily removed. However, installing a distillation tower not only implies high capital costs, but also expensive operating costs. Other processes like liquid adsorption and membrane separation, are also expensive and do not remove carbon monoxide to the necessary level for processes, such as metallocene polymerisations.

Other methods include using molecular sieves to remove carbon monoxide. An example is provided in US 4,717,398, which claims a pressure swing process for selectively adsorbing an organic gas containing unsaturated linkages, such as carbon monoxide, from a mixture of gases by passing the mixture over a zeolite ion-exchanged with cuprous ions. However, this particular process is unsuitable for separating carbon monoxide from hydrocarbon mixtures containing olefins, since the zeolite also adsorbs olefins.

Other molecular sieves have also been used to selectively adsorb carbon monoxide by chemisorption. For example, US 4,019,879 and US 4,034,065 describe the use of high silica zeolites, which can selectively adsorb carbon monoxide. The problem, however, is that there is only a moderate capacity for carbon monoxide. In addition, very low vacuum pressures must be applied for regenerating the zeolite.

Another example of a molecular sieve is given in US 2005/0137443. This document discloses the use of a modified clinoptilolite for the adsorption of carbon monoxide from hydrocarbon streams. It states that the process can remove at least 90% of carbon monoxide from hydrocarbon feeds, which initially contained at least 5 ppm of carbon monoxide. It does not sufficiently reduce the carbon monoxide content.

US 2003/0105376 discloses the purification of polyolefins using a combination of two heterogeneous adsorbents. The second adsorbent is claimed to remove carbon monoxide and comprises cations of at least one element selected from various metals. The document discloses that a feedstock containing less than 5ppm of carbon monoxide can be obtained. However, this is still well above the acceptable level necessary, for example, for metallocene catalyst polymerisations.

US 2005/0247196 provides a method for purifying unsaturated hydrocarbons to be used as a matrix gas for low-level calibration standards. The system comprises at least three purification cartridges, two of which comprise metal oxides and one of which comprises a molecular sieve. The best example given shows that propylene, which had an initial concentration of 0.796 ppm, was purified with this system to provide a hydrocarbon gas with a final carbon monoxide concentration of 0.008 ppm. Despite this promising result, the process is complicated, costly and time consuming. For example, the carbon monoxide-containing hydrocarbon feedstock has to be passed through the cartridges at a temperature in excess of 150°C. This is not a process suitable for largescale use. What is needed is an efficient process that does not require high temperatures.

GB 1324826 refers to carbon monoxide removal for efficient olefin polymerisation using copper in a mixture of cupric and cuprous state. It is claimed that to prevent serious catalyst poisoning during polymerisation less than 0.2 ppm of carbon monoxide must be present in the hydrocarbon feedstocks. However, recent studies show that this level of purity is insufficient for processes, such as metallocene catalyst polymerisation.

US 2005/0241478 discloses the adsorption of carbon monoxide using an adsorbent composed of copper, zinc and zirconium, but it does not disclose the efficiency with which the carbon monoxide is removed. It is indicated that oxygen is also adsorbed during the process. In general, oxygen impurities in an olefin feedstock pose fewer problems for catalysts, and therefore do not necessarily need to be removed. Since the carbon monoxide has to compete with oxygen for empty sites on the adsorbent, it is suspected that this cannot be a very efficient process for carbon monoxide removal.

WO 95/21146 teaches a process for removing carbon monoxide and arsine by contacting the hydrocarbon stream with a sorbent comprising various combinations of copper in different oxidation states and manganese dioxide. However, space velocities are very low and therefore this process only works efficiently with larger sorbent beds. For smaller beds, higher hourly space velocities are desirable in order to make up for the lower volume of catalyst available. Hence, a sorbent material that can still remove carbon monoxide even at high space velocities is needed.

EP 0 537 628 claims a catalyst system for propylene purification using a combination of various metal oxides, in particular a mixture of copper oxide and chromium oxide. This system can reduce the carbon monoxide concentration to below 30ppb. However, it should be pointed out that the carbon monoxide is not removed, but merely transformed into carbon dioxide. To remove the carbon dioxide an additional bed of calcium carbonated or activated carbon is necessary.

JP-05070373-A2 discloses the simultaneous removal of carbon monoxide and carbonyl sulphide using metallic nickel. After treatment, the hydrocarbon can contain 0.1 ppm or less of carbon monoxide. Hence, this process also does not remove carbon monoxide sufficiently enough.

Accordingly, it can be seen that there is a need for a more efficient process to reduce the carbon monoxide concentration in olefin-containing hydrocarbon feedstocks to several ppb.

It is hence an aim of the invention to provide an efficient process to reduce carbon monoxide concentrations in olefin-containing hydrocarbon feedstocks to several ppb.

It is another aim of the invention to provide an efficient process to reduce carbon monoxide concentrations in propylene-containing hydrocarbon feedstocks to several ppb.

It is also an aim of the invention to provide a process to reduce carbon monoxide concentrations in olefin-containing hydrocarbon feedstocks, which can be carried out at high space velocities.

It is furthermore an aim of the invention to provide a process to reduce carbon monoxide concentrations in olefin-containing hydrocarbon feedstocks, which can be carried out at temperatures below 40°C.

### SUMMARY OF THE INVENTION

The present invention is directed to a process for the reduction of carbon monoxide in olefin-containing hydrocarbon feedstocks. In particular, the present invention is a process for removing carbon monoxide from an olefin-containing hydrocarbon feedstock, comprising the steps of:
a. passing the said hydrocarbon feedstock over a material comprising nickel deposited on a support material wherein said nickel is present as both nickel oxide and metallic nickel.
b. recovering a hydrocarbon stream having a substantially reduced carbon monoxide content.

### DETAILED DESCRIPTION OF THE INVENTION

The present invention relates to the removal of carbon monoxide from olefin-containing hydrocarbon streams onto a sorbent. While the subsequent discussion will describe the invention in terms of treating propylene-containing feeds, the present invention may be applicable to the treatment of hydrocarbon feeds containing other olefins i.e. ethylene, propylene, butenes, pentenes, hexenes, octenes or any combinations thereof. However, due to propylene's physical properties, the process is preferably applied to the removal of carbon monoxide from propylene.

For the purposes of the present invention, the nickel/nickel oxide material is referred to generally as a sorbent material and the carbon monoxide as a sorbate. The exact mechanism of the process (i.e. adsorption or absorption) is irrelevant as to the result.

The sorbent material of the present invention comprises nickel deposited on a support material, the nickel being present both as metallic nickel and as nickel oxide. Silica, silico-aluminas, alumina, kieselguhr, zeolites and other similar materials, whether amorphous or crystalline, can be utilised as the support. The total weight of nickel and nickel oxide may represent up to about 80 wt.% of the sorbent material, with the provision that metallic nickel should not represent less than 10 wt.%, nor more than 50 wt.% of the sorbent. Preferably, the weight ratio of metallic nickel to nickel oxide is of about 0.4 to about 2.0, and the sorbent comprises from about 30 to about 60 wt.% of support material. When carrying out the process of the invention with a sorbent material outside this definition, the results obtained may no longer be satisfactory, although some carbon monoxide will still be removed. Whilst not wishing to be bound by any theory, the Applicant believes that larger crystallites are formed if the nickel to nickel oxide ratio is higher, thus leading to a lower efficiency; similarly, an excessive total nickel content tends to lower the specific surface and consequently the efficiency, while a too low total nickel content would lead to an insufficient capacity for sorbing carbon monoxide.

The nickel can be deposited on the support by any of the several methods well known to those skilled in the art. For example, nickel can be deposited on the support by dissolving nickel nitrate in water, mixing the solution with the support and precipitating the nickel, for example in the form of nickel carbonate, and subsequently washing, drying and calcining the precipitate. The nickel deposited in this manner is then partially reduced by means of hydrogen to form metallic nickel, the remainder being in the form of nickel oxide.

In general, the size of the nickel crystallites after reduction is from 1 to 2 nm. The size of the nickel crystallites depends on the extent of reduction carried out. In fact, if the degree of reduction is increased, the size of the crystallites is increased but the sorbent material obtained does not have the desired properties. On the other hand, if the degree of reduction is too low, the crystallites still have good dimensions but the quantity of nickel available in this case is too small to ensure successful purification of the feedstock.

The specific surface area of the sorbent material obtained after reduction is generally between 100 and 200 m²/g.

The particle size of the sorbent material depends especially on the pressure drop allowed in the reactor; it has been noted, however, that it is advantageous to use the sorbent material in finely divided form. Preferably, the particle diameter of this material when spherical does not exceed about 3.5 mm and is most preferably from 1 to 2.5 mm. When cylindrical particles are used, they preferably have a diameter of from 1 to 2 mm and a length of from 3 to 8 mm. Trilobes of similar size may also be used.

The sorbent material is usually prepared ex situ and stored either under a convenient saturated liquid hydrocarbon, like cyclohexane or dodecane, or under a non-oxidizing atmosphere like N₂. It can also be protected by deposition of a carbon dioxide layer on the surface, said layer protecting the sorbent material from air, thus allowing easy handling.

It has been found that propylene adsorbs onto the sorbent material when contacted with the feedstocks, and that the propylene adsorption reaction, occurring during startup, is exothermic. Under certain conditions, and particularly when the sorbent material is stored under a non-oxidizing atmosphere, the temperature rise may be very important. More particularly the temperature at the surface of the material may be much higher than that measured with a thermocouple, and the sorbent material may thus be damaged. In addition, the high temperatures trigger undesired side-reactions, more particularly propylene dimerisation and trimerisation. The dimers are hexenes, which can copolymerise with propylene. If this purified propylene stream is then used for polymerisations, these by-products can break the regularity of the linear chain of, for example, isotactic polypropylene or otherwise cause other process difficulties. As a result, the copolymer has a lower crystallinity than polypropylene, and thus a lower melting point; its mechanical resistance is also lower. More seriously, during polymerisation these dimers also act as retarders by blocking active sites on the catalyst, thereby significantly reducing productivity. Also, such impurities typically reduce the polymerisation rate of propylene polymerisations thus leading to reduced economic advantage.

The Applicants have found that an excessive increase in the temperature of the sorbent material can be avoided by conditioning it before use. Conditioning can be carried out by passing an inert gas flow containing a minor amount of at least one light olefin, preferably propylene, in a concentration of from 0.1 to 5 vol %, over said material. The inert gas is usually nitrogen, which should contain the least possible amount of oxygen. It is preferable to begin the conditioning procedure by passing essentially pure inert gas over the material. The conditioning step is preferably carried out at about atmospheric pressure, at or below ambient temperature. It is continued until the propylene concentration at the outlet equals that introduced. It is also possible to monitor the passage of an exotherm, shown by thermocouples introduced within the sorbent material.

It is known that, when the sorbent material is prepared ex situ and protected by a monolayer of carbon dioxide (believed to be sorbed onto the nickel surface), the sorbent material must be pre-treated prior to its conditioning by passing an initial inert gas (containing the least possible amount of oxygen) over it at a temperature of from about 150°C to about 350°C, preferably at about 250°C and preferably at about atmospheric pressure. This is then preferably followed by passing a mixture of inert gas and hydrogen containing an increasing concentration of hydrogen over it (to remove any oxygen possibly that may have been sorbed despite all precautions), before purging it free of hydrogen with an inert gas flow at about 250°C.

The process of the present invention is capable of reducing the carbon monoxide concentration in the treated hydrocarbon feedstock to several ppb. The original carbon monoxide concentration may be as high as 1000 ppm or higher depending on the process used to produce the original feedstock. However, in such cases, it is usually more economical to carry out in advance another known purification process such as distillation, catalytic oxidation with oxygen to carbon dioxide or the use of molecular sieves to reduce the carbon monoxide concentration to 100 ppm or less.

Once spent, the sorbent can be partially reactivated by treatment with inert gas at elevated temperatures and optionally with addition of hydrogen.

Optionally, additional sorbents may be used in combination with the nickel sorbent to remove other impurities that may be present in the feedstock. The feedstock may be passed over one or more additional sorbents prior to passing it over the nickel sorbent. These act as guard beds and as a result, the nickel sorbent's overall lifetime is increased. One or more additional sorbents can also be used after the nickel sorbent bed. The additional sorbents can be any sorbent known to a person skilled in the art. Examples of possible additional sorbents are metal oxides such as copper oxide, zinc oxide, zirconium oxide or manganese oxide, aluminas (including promoted aluminas), palladium, platinum, and molecular sieves such as 3A, 4A, 5A or 13X, as well as copper/copper oxide sorbents. Preferably, molecular sieve 13X is used, because of its larger pore size.

In polyolefin production, the hydrocarbon feedstock generally comprises more than 75 wt.% of olefins, more particularly from 85 to 99.99 wt.%. In one embodiment of the present invention, the olefin-containing hydrocarbon feedstock is passed over the sorbent material at a temperature of from -10°C to 80°C, preferably of from 0°C to 40°C, more preferably 0°C to 20°C and at a liquid hourly space velocity (LHSV) of from 0.1 to 60 l/l.h, preferably about 30 l/l.h. It is surprising that even at temperatures as low as 0°C to 20°C and at LHSVs of around 30l/l.h, the nickel sorbent is still capable of reducing the carbon monoxide content to several ppb. Thus the liquid hourly space velocity can be increased to make up for a smaller sorbent volume i.e. smaller vessels or driers containing the nickel sorbent.

If carrying out the process with a propylene-containing feedstock, the pressure used is generally such as to retain the feedstock in liquid phase.

In utilising the latest generation of metallocene-type catalysts in the production of polypropylene and polyethylene, it is essential that the hydrocarbon feedstock contains less than 20 ppb, preferably less than 10 ppb, more preferably less than 3 ppb, most preferably less than 1ppb of carbon monoxide. It has been unexpectedly found that by passing the hydrocarbon feedstock over a sorbent material as herein before described, the feedstock obtained can have content of a carbon monoxide content below 20ppb, preferably below 10ppb, more preferably below 5ppb. This result is unexpected due to the degree of purity obtained and due to the fact that this process can be carried out either in the presence or in the absence of water. In addition, the invention can be rapidly carried out due the high liquid hourly velocities of the propylene that can be used when passing it over the sorbent material.

Furthermore, it has been found that the olefin-containing hydrocarbons purified according to the present invention, result in higher catalyst productivity during metallocene-type polymerisations. This results in a considerable reduction in the amount of used catalyst. In addition, the catalyst productivities remain more constant, allowing for better control over the polymer products and more stable melt flow indices.

The examples, which follow, are given to provide a better illustration of the process of the present invention. These examples should not, however, be construed as limiting the scope of the invention as there are many variations which may be made thereon, as those skilled in the art will recognise.

### EXAMPLES OF THE INVENTION

### Example 1

To determine whether the sorbent removes carbon monoxide, a sample of propylene enriched with carbon monoxide was collected at the top of a propylene / propane / ethane splitter. Measurements were made which indicated that carbon monoxide levels were between 1200 and 1400 ppm. The carbon monoxide enriched propylene was passed through a 3.8 litre column containing the nickel-nickel oxide sorbent at room temperature at a LHSV of 30 l/l.h. The carbon monoxide concentration at the outlet of the column was found to be substantially lower than before treatment with the sorbent, down to 20-30ppb. The test results indicate that the sorbent in the invented method is capable of reducing the carbon monoxide content in the propylene to very low levels.

### Examples 2-4

A polymerisation was performed using a standard metallocene isotactic polypropylene (miPP) catalyst described in US 6,855,783. A 3-litre reactor was charged with 1.5 litres of propylene and 0.4 NL of hydrogen. The catalyst (0.3 ml of 20wt% catalyst slurried in oil) was brought into contact with 69 mg of TEAL for 5 minutes and charged into the reactor with the addition of 0.5 litres of propylene. The polymerisation temperature was maintained at 70°C for 1 hour, after which, the contents of the reactor were flared and the polymer isolated. Propylene used in this study was either directly injected into the reactor or first passed through a purifier containing nickel and nickel oxide. The results of the polymerisation with and without passage through the nickel purifier are shown in Table 1. The productivity is provided in relative terms. The results indicate that with the same catalyst injected into the reactor in the same amounts, the productivity increases 33-34 fold when the propylene is passed through the nickel - nickel oxide purifier prior to polymerisation.

**Table 1**

| **Example** | **Sorbent** | **Yield** / **g** | **Relative Productivity** |
|---|---|---|---|
| 2 | Nickel / Nickel oxide | 480 | 34 |
| 3 | Nickel / Nickel oxide | 468 | 33 |
| 4 | None | 14 | 1 |

## Claims

1. A process for removing carbon monoxide from an olefin-containing hydrocarbon feedstock comprising the steps of:
a. passing the said hydrocarbon feedstock over a material comprising nickel deposited on a support material wherein said nickel is present as both nickel oxide and metallic nickel.
b. recovering a hydrocarbon stream having a substantially reduced carbon monoxide content.

2. The process according to claim 1, wherein the total weight of nickel oxide and metallic nickel represents from 10 to 80 wt.% of the sorbent material, and the sorbent material includes 20 to 90 wt.% of the support material.

3. The process according to claim 1 or 2, wherein the weight ratio of metallic nickel to nickel oxide is from 0.4 to 2.0, with the provision that metallic nickel should neither represent less than 6 wt.%, nor more than 50 wt.% of the sorbent material, and the sorbent material comprises from 40 to 70 wt.% of metallic nickel and nickel oxide and from 30 to 60 wt.% of support material.

4. The process according to any one of the preceding claims, wherein said sorbent material has a specific surface area from 100 to 200 m²/g.

5. The process according to any one of the preceding claims, wherein the feedstock comprises more than 75 wt.% of propylene, preferably from 85 to 99.99 wt.%.

6. The process according to any one of the preceding claims, carried out at a temperature of from -10°C to 80°C and at a liquid hourly space velocity (LHSV) from 0.1 to 60 l/l.h.

7. The process according to claim 6, wherein the temperature is from 0°C to 20°C and the LHSV is around 30 l/l.h.

8. The process according to any one of the preceding claims, wherein the hydrocarbon feedstock comprises up to 100 parts per million (ppm) by weight of carbon monoxide.

9. The process according to any one of the preceding claims, wherein at any stage prior to step (b), the olefin-containing hydrocarbon feedstock is passed over one or more of the following:
- molecular sieves, preferably 13X molecular sieves
- metal oxides
- alumina
